# EUROPÄISCHE PATENTSCHRIFT

(11) **EP 3 342 441 B1**
(45) Veröffentlichungstag und Bekanntmachung des Hinweises auf die Patenterteilung: **31.08.2022**
(21) Anmeldenummer: 17201549.7
(22) Anmeldetag: 14.11.2017
(51) Int. Cl.: A61M 5/34, A61M 5/31

(54) **SPRITZE MIT UNTERSCHIEDLICHEN MATERIALIEN**
SYRINGE HAVING DIFFERENT MATERIALS
SERINGUE COMPRENANT DE DIFFÉRENTS MATÉRIAUX

(30) Priorität: 02.01.2017 DE 102017200007
(43) Veröffentlichungstag der Anmeldung: 04.07.2018
(73) Patentinhaber: SCHOTT AG, 55122 Mainz (DE)
(72) Erfinder: Auerbach, Judith, 9052 Niederteufen (CH); FISCHER, Bastian, 9000 St. Gallen (CH); DERKSEN, Helena, 9014 St. Gallen (CH)
(74) Vertreter: Sawodny, Michael-Wolfgang

(56) Entgegenhaltungen:
- JP-A- 2006 166 961
- JP-A- 2008 246 070
- US-A1- 2006 106 349
- US-A1- 2014 025 017

## Beschreibung

Die Erfindung betrifft eine Spritze mit einem Spritzenkörper sowie einem Spritzenkonus, der eine distale Öffnung umfasst und einen, im Bereich des Spritzenkonus angeordneten Anschluss, insbesondere in Form eines Gewindes, wobei der Spritzenkörper aus einem ersten Material und der Anschluss aus einem zweiten Material besteht.

Aus einer Vielzahl von Patenten sind Spritzen bekannt geworden, die einen Anschluss für unterschiedliche Konnektoren, die manuell auf die Spitze der Spritze montiert werden, aufweisen.

Eine derartige Spritze zeigt bspw. die US 2013/ 079 276 A. Aus der US 2013/079 276 A ist ein Spritzenkörper bekannt geworden, bei dem eine Transportkappe, ein intravenöser Port oder eine Injektionsnadel in ein Gewinde oberhalb des Spritzenkonus eingeschraubt werden kann. Wird bspw. eine Transportkappe in das Gewinde eingeschraubt, so kann der Spritzenkonus geschützt werden.

Aus der DE 202 158 07 U1 ist eine Spritze mit einem Gewinde im Bereich des Luer-Lock-Anschlusses bekannt geworden, wobei das Gewinde, Teil des Spritzenkörpers ist. Der Luer-Lock-Anschluss gemäß der DE 202 158 07 U1 weist ein Innengewinde auf, das im Bereich des Spritzenkonus angeordnet ist.

Die DE 692 26 166 T2 zeigt ebenfalls eine Kunststoffspritze, wobei das Nadelanschlussstück die Form eines standardmäßigen Luer-Lock-Anschlusses mit einem Luer-Kegel und einer Außenwand mit einem Schraubgewinde um ihre innere Umfangswand aufweist.

Eine ähnliche Anordnung ist auch aus der
EP 1 080 742 A1 oder der EP 1 410 819 A1 bekannt geworden. Sowohl bei der EP 1 080 742 A1 wie bei der EP 1 410 819 A1 ist das Gewinde, das als Anschluss für Konnektoren dient, ein Teil des Luer-Konus. Ähnliche Systeme, bei denen Teile des Luer-Konus mit einem Innengewinde des Anschluss für Konnektoren bzw. Spritzennadeln etc. vorgesehen sind, zeigen die EP 1 923 086 A1 und die EP 3 042 689 A1.

Aus der WO 2012/116 790 A1 ist eine Spritze mit einem Spritzenzylinder und einem an einem distalen Ende des Spritzenzylinders vorgesehene Nadelansatzstück bekannt geworden, wobei die Spritze einen Verschluss aufweist, der eine das Nadelansatzstück dichtend verschießende Verschlusskappe und eine Sicherungskappe umfasst, wobei die Sicherungskappe die Verschlusskappe umgreift und über einen Haltering an dem Nadelansatzstück befestigt ist. Die Spritze zeichnet sich dadurch aus, dass zumindest die Sicherungskappe und die Verschlusskappe einstückig ausgebildet sind.

Zwar ist in der WO 2012/116 790 A1 die Rede davon, dass der Haltering aus einem thermoplastischen Elastomer (TPE) oder aus Polypropylen wie auch die Verschlusskappe und der Sicherungsring gefertigt sein kann. An der anderen Seite umfasst das Nadelansatzstück bevorzugt Glas bzw. besteht aus diesem. Eine Ausgestaltung des Nadelansatzstückes sowie der Spritze aus einem harten Kunststoffmaterial, umfassend ein Cycloolefincopolymer (COC) oder Cycloolefinpolymer (COP), zeigt die WO 2012/116 790 A1 nicht.

Bei der Verwendung von COC oder COP als Material für den Spritzenzylinder besteht das Problem, dass mechanische Spannungen zwischen dem Anschluss bzw. dem Gewinde und dem in das Gewinde eingeschraubten Gegengewinde, das dem Konnektor zugeordnet ist, Risse im Material des Spritzenkörpers entstehen können, wodurch die Funktionalität der Spritze beeinträchtigt wird. Im schlimmsten Fall führen derartige mechanische Spannungen zu Beschädigungen der Spritze selbst oder deren Undichtigkeit. Dies ist beispielsweise dann der Fall, wenn als Material des Spritzenkörpers COC (Cycloolefincopolymer) oder COP (Cycloolefinpolymer) gewählt werden.

Zwar zeigen die US 8,038,182 B1 und die EP 0 838 229 A2 auch Spritzenkörper aus COC. Um eine Beschädigung des Spritzenkörpers aus COC zu vermeiden, schlägt die US 8,038,182 B2 eine aufwändige Konstruktion des Anschlusses, der in das Gewinde des Luer-Lock eingebracht wird, vor, bei dem nach oben und nach unten gerichtete Kräfte im Anschlussgewinde dazu führen, dass sich der Anschluss in einen Freiraum ausdehnt und radiale Kräfte auf den Spritzenkörper, die zu einem Bruch führen könnten, vermieden werden. Bei der EP 0 838 229 A2 wird ein Bruch durch Einbringen eines Halteelements verhindert.

Nachteilig an sämtlichen Ausgestaltungen gemäß dem Stand der Technik war, dass das Anschlussstück, bspw. das Gewinde des Luer-Lock dasselbe Material aufwies, wie die Spritze selbst. Dies hat dazu geführt, dass mechanische Spannungen zwischen dem Anschluss, bspw. dem Gewinde und dem in das Gewinde eingeschraubten Gegengewinde, das dem Konnektor zugeordnet ist, zu Rissen im Material des Spritzenkörpers geführt haben, wodurch die Funktionalität der Spritze beeinträchtigt wird. Im schlimmsten Fall können derartige mechanische Spannungen zu Beschädigungen der Spritze selbst und zu deren Undichtigkeit führen. Dies war insbesondere dann der Fall wenn das Material des Spritzenkörpers ein hartes Material war, beispielsweise COC (Cycloolefin Copolymer) oder COP (Cycloolefinpolymer und die Konnektoren aus einem anderen Material wie PC (Polycarbonate), PVC (Polyvenylchlorid), PE (Polyethylen) oder PA (Polyamid) bestanden.

Ist der gesamte Spritzenkörper aus einem weicheren Kunststoffmaterial, um die Rissproblematik zu vermindern, so ergibt sich das Problem, dass zwar ein Spritzenkörper aus Polypropylen (PP) die Spannungen durch die Konnektoren aufgrund der Materialeigenschaften kompensieren kann, jedoch kann in einem Spritzenkörper aus Polypropylen (PP) ein pharmazeutisches Medium aufgrund des Diffusionsverhaltens nicht über mehrere Monate gelagert werden.

Ein weiteres Problem besteht darin, dass Konnektoren, die bspw. in das Gewinde des Luer-Locks eingeschraubt werden sollen, eine Vielzahl unterschiedlicher Materialien wie PC, PVC, Polyethylen (PE) umfassen, die mit dem Material des Gewindes des Luer-Locks verbunden werden. Ein weiteres Problem, der derzeitigen Konnektoren, ist darin zu sehen, dass sie in der Regel manuell in das Gewinde eingedreht werden und das Eindrehmoment über einen breiten Bereich von beispielsweise 12Ncm bis 56Ncm bevorzugt 12Ncm bis 80Ncm schwanken kann. Diese Spannungen führen dazu, dass entweder am Gewinde oder am Konnektor Risse entstehen. Im schlimmsten Fall können sogar Brüche aufgrund dieser Spannungen entstehen. US2014025017 beschreibt eine Spritze mit einem Spritzenkörper sowie einem Spritzenkonus, der eine distale Öffnung umfasst und einen im Bereich des Spritzenkonus angeordneten Anschluss, insbesondere ein Gewinde, wobei der Spritzenkörper aus einem ersten Material besteht und der Anschluss insbesondere das Gewinde aus einem zweiten Material. Das erste Material ist verschieden zum zweiten Material und das zweite Material ist ein weicheres Material als das erste Material. Das erste Material ist ein Cycloolefincopolymer (COC) oder ein Cycloolefinpolymer (COP).

Aufgabe der Erfindung ist es daher, die Nachteile des Standes der Technik zu überwinden und insbesondere, eine Spritze anzugeben, die das Aufbringen, bzw. den Anschluss unterschiedlichster Konnektoren ermöglicht, ohne das Defekte am Material des Spritzenkörpers auftreten.

Erfindungsgemäß wird diese Aufgabe durch eine Spritze mit den Merkmalen des Anspruches 1 gelöst.

Bei einem erfindungsgemäßen Spritzenkörper mit einem Spritzenkonus der eine distale Öffnung umfasst und einen in Bereich des Spritzenkonus angeordneten Anschluss sind die Materialien von Anschluss und Spritzenkörper unterschiedlich. Dabei besteht der Spritzenkörper aus einem ersten Material und der Anschluss, insbesondere das Gewinde, wenigstens abschnittsweise aus einem zweiten Material. Erfindungsgemäß ist das zweite Material ein weicheres Material als das erste Material. Das zweite Material kann ein thermoplastisches Elastomer (TPE), ein Elastomer oder Polypropylen (PP), Polycarbonat (PC), Polyethylen (PE), Polyamid (PA) oder COC-E sein, das sich dadurch auszeichnet, dass es eine höhere Kerbschlagfestigkeit und/oder einen geringeren

E-Modul aufweist. Das zweite weiche Material wird bevorzugt dort eingesetzt, wo beim Einschrauben beispielsweise von Konnektoren in den Anschluss hohe Kräfte auftreten. Dann ist auch eine abschnittsweise Ausbildung möglich an dem Orten der hohen Kräfte.

In vorliegender Anmeldung wird unter einem weicheren Material, ein Material verstanden, dessen E-Modul um 10 bis 60 % geringer ist als derjenige des harten Materials. Bevorzugt liegt der E-Modul des ersten harten Materials im Bereich von größer 2500 bis 3500 MPa, bevorzugt 2700 bis 3200 MPa, und der des weicheren zweiten Materials im Bereich von1200 bis 2500 MPa, bevorzugt 1500 bis 1800 MPa.

Des Weiteren zeichnet sich das zweite weiche Material durch eine hohe Kerbschlagfestigkeit größer 2kJ/m² aus.

Unter Kerbschlagfestigkeit wird ein Maß für die Widerstandsfähigkeit eines Werkstoffs gegen eine schlagartige (dynamische) Beanspruchung verstanden. Die Einheit ist die geleistete Kerbschlagarbeit bezogen auf die Bruchfläche in Joule pro Flächeneinheit [J/cm²]. Generell haben weiche Materialien eine hohe Kerbschlagfestigkeit, wohingegen harte Materialien, Materialien mit niedriger Kerbschlagfestigkeit sind. Sprödere Materialien wie COC haben eine Kerbschlagfestigkeit kleiner 2kJ/m².

Dadurch, dass im Bereich des Anschlusses, d.h. insbesondere des Gewindes, ein weicheres Material verwendet wird, als für den Spritzenkörper selbst, ist es möglich, dass das weichere Material Belastungen und Kraftspitzen, die beim Einschrauben eines Konnektors oder Adapters in das Gewinde auftreten können, auffängt und diese nicht an den Spritzenkörper weitergegeben werden. Durch die Verwendung eines weichen Kunststoffmaterials ist es möglich, unterschiedliche Belastungsfälle zu kompensieren, wie bspw. Kraftspitzen, Drehmomente, etc. Bevorzugt weist der Anschluss in der Geometrie die Geometrie eines Luer-Lock-Adapters (LLA) auf. Dies stellt sicher, dass eine Standardspritze zur Verfügung gestellt wird, die mit den unterschiedlichsten Anschlusselementen insbesondere Konnektoren und Adaptern zusammenwirken kann.

Das weichere Material im Bereich des Anschlusses, insbesondere des Gewindes, kann zwar nicht verhindern, dass mechanische Belastungen auftreten, jedoch stellt das weichere Material einen Puffer zur Verfügung, um Kraftspitzen bspw. beim Eindrehen des Konnektors abzufangen und gleichmäßig über den Spritzenkörper zu verteilen. Das weiche Material stellt somit eine Art Puffer dar. Möglich ist es, dass das weiche Material sowohl die Gewindegeometrie darstellt, als auch zwischen das Gewinde des Konnektors und das Gewinde des Spritzenkörpers, der als Anschluss dient, als Puffer eingebracht wird.

Das erste, harte Material des Spritzenkörpers ist bevorzugt ein Cycloolefincopolymer (COC) oder ein Cycloolefinpolymere (COP). Der Vorteil der Verwendung des Cycloolefincopolymer oder des Cycloolefinpolymere ist, dass diese Materialien eine hohe Wasserdampfbarriere darstellen, die es ermöglicht, Flüssigkeiten, insbesondere flüssige Arzneimittel über einen längeren Zeitraum, der mehrere Jahre betragen kann, ohne bzw. mit sehr geringem Volumenverlust zu lagern.

Die Erfinder haben herausgefunden, dass dann wenn COC und COP als Materialien für den Spritzenkörper verwendet werden, die Problelmatik der Rissbildung verhindert werden kann, wenn der Anschluss für die Konnektoren, bspw. der Luer-Lock aus einem weicheren Material als der Spritzenkörper gefertigt ist. Durch die Verwendung eines weicheren Materials kann erreicht werden, dass auch bei hohen Eindrehmomenten Spannungen am Gewinde oder am Konnektor und damit Brüche aufgrund dieser Spannungen in der Spritze vermieden werden.

Als weicheres, zweites Material wird bevorzugt ein Elastomer, insbesondere ein thermoplastisches Elastomer (TPE) oder COC-E verwendet. Andere mögliche Polymere können auch Polypropylen (PP), Polyethylen (PE), Polycarbonat (PC), Polyamid (PA) sein.

Um die Spritze im Bereich von pharmazeutischen Produkten einsetzen zu können, ist es notwendig, dass sowohl das erste, wie auch das zweite Material des Spritzenkörpers bzw. des Anschlusses, Materialien umfasst, die bei Temperaturen > 100°C, insbesondere 121°C, insbesondere maximal 180°C, sterilisierbar sind. Beispiele für derartige Materialien sind für den Spritzenkörper COC oder COP und für den Konnektor COC-E, TPE, PP, PE, PC oder PA.

Der Spritzenkörper stellt ein erstes Bauteil der Spritze dar und der Anschluss, insbesondere das Gewinde und unter Umstände auch der Konus, ein zweites Bauteil. Erstes Bauteil und zweites Bauteil sind getrennte Bauteile, die stoffschlüssig miteinander verbunden sind, z.B. durch Schweißen.

Besonders bevorzugt ist es, wenn die Verbindung zwischen Spritzenkörper und Anschluss derart ausgestaltet ist, dass das erste Bauteil mit dem zweiten Bauteil zwar verbunden ist, diese Verbindung aber bei einer vorbestimmten Kraft, gelöst werden kann, d.h. das erste Bauteil mit dem zweiten Bauteil definiert lösbar verbunden ist. Bei einer derartigen Anordnung wird bei eindeutiger Überbelastung ein sichtbarer Bruch an einer definierten Stelle ausgelöst anstatt an einer kritischen Position, z.B. Dichtstelle, die nicht eingesehen werden kann.

Bei der Herstellung einer erfindungsgemäßen Spritze sind unterschiedliche Möglichkeiten denkbar. Ein erstes Verfahren zeichnet sich dadurch aus, dass der Spritzenkörper aus einem ersten Material und der Anschluss, insbesondere das Gewinde, aus einem zweiten Material ist, wobei das zweite Material weicher als das erste Material ist, und erstes und zweites Material getrennt zur Verfügung gestellt werden. In einem weiteren Verfahrensschritt werden der Anschluss und der Spritzenkörper miteinander verbunden, und zwar stoffschlüssig. Besonders bevorzugt ist bei einem stoffschlüssigen Verbund wenn Spritzenkörper und Anschluss durch Schweißen, insbesondere Ultraschall-Schweißen verbunden werden.

Alternativ zu dem erstgenannten Herstellverfahren, bei dem jede Komponente einzeln hergestellt wird, ist es auch möglich, dass gesamte Bauteil also die Spritze mit Spritzenkörper und Anschluss durch Mehrkomponenten-Spritzgießen herzustellen. Beim Mehrkomponenten-Spritzgießen wird ein Spritzgussteil erhalten, das aus zwei oder mehreren unterschiedlichen Kunststoffen besteht. Beim Mehrkomponenten-Spritzgießen sind unterschiedliche Verfahren denkbar. Eine Spritzgießmaschine für Mehrkomponenten-Spritzgießen umfasst zwei oder mehreren Spritzeinheiten aber nur eine Schließeinheit. Zudem kann auch ein vorgespritztes Teil in ein Werkzeug zur Umspritzung eingelegt werden. Beim Mehrkomponenten-Spritzgießen können die entsprechenden Bauteile kostengünstig mit nur einem Werkzeug in einem Arbeitsgang hergestellt werden. Zudem können durch Mehrkomponenten-Spritzgießen geringere Lagetoleranzen umgesetzt werden als bei der Montage.

Mit dem Mehrkomponenten-Spritzgießen ist es möglich, dass das weiche Material an das harte Material oder umgekehrt angespritzt wird. Dies erspart den Vorgang der Montage und führt zu einer physikalischen Anbindung zwischen dem harten und weichen Material. Beim Mehrkomponenten-Spritzgießen ist es möglich, dass zwischen dem weichen und dem harten Material die Anbindung eingestellt werden kann, so dass der Anschluss lösbar auf dem Spritzenkörper angebracht wird.

Besonders bevorzugt ist es, wenn das weichere Material ein erhöhten Reibungskoeffizienten aufweist, der dazu führt, dass das Gewinde nicht überdreht werden kann. Bei der Anbindung des ersten an das zweite Material sollte die Verbindungsfläche so ausführbar sein, dass ein möglichst großer Oberflächenkontakt der beiden Materialien die Stabilität der Verbindung gewährleistet.

Die Erfindung soll nachfolgend anhand der Figuren beispielhaft beschrieben werden. Es zeigen:
- Fig. 1a-1c: eine erste Version einer mechanischen Befestigung des Anschlusses am Spritzenkörper (nicht erfindungsgemäß)
- Fig.2a-2c: eine zweite Version einer mechanischen Befestigung des Anschlusses am Spritzenkörper (nicht erfindungsgemäß)
- Fig.3a1-3c: eine Version mit Spritzenkörper und Anschluss als Einzelteile, wobei die Einzelteile Ultraschall verschweißt sind
- Fig.4a-4b: erste Ausführungsform eines Anschlusses und Spritzenkörper erstellt mittels Mehrkomponenten-Spritzgießen
- Fig. 5a-5d: zweite Ausführungsform eines Anschlusses und Spritzenkörpers erstellt mittels Mehrkomponenten Spritzgießen

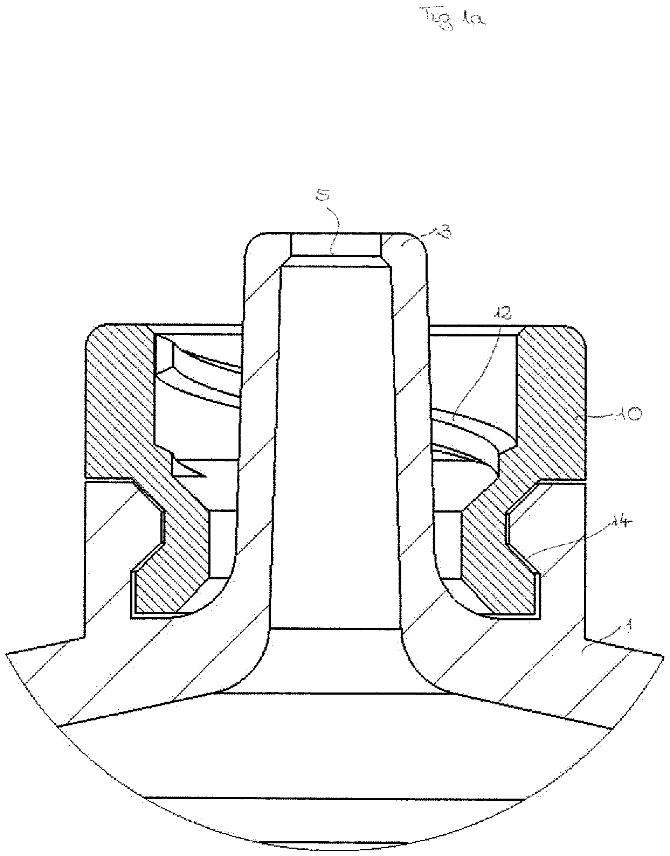

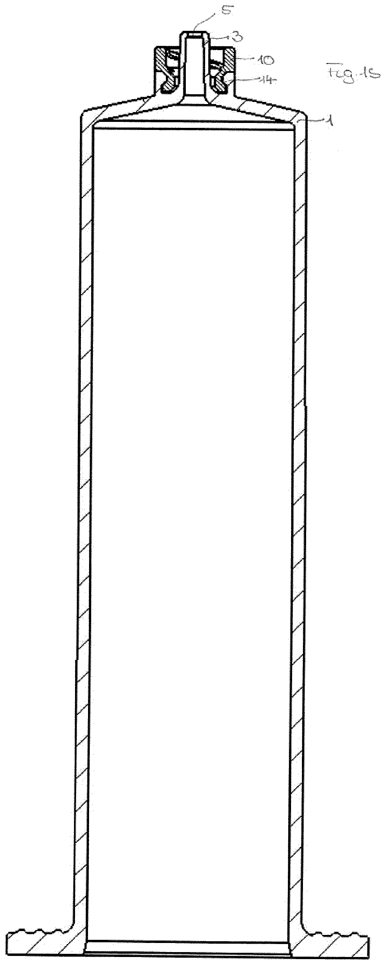

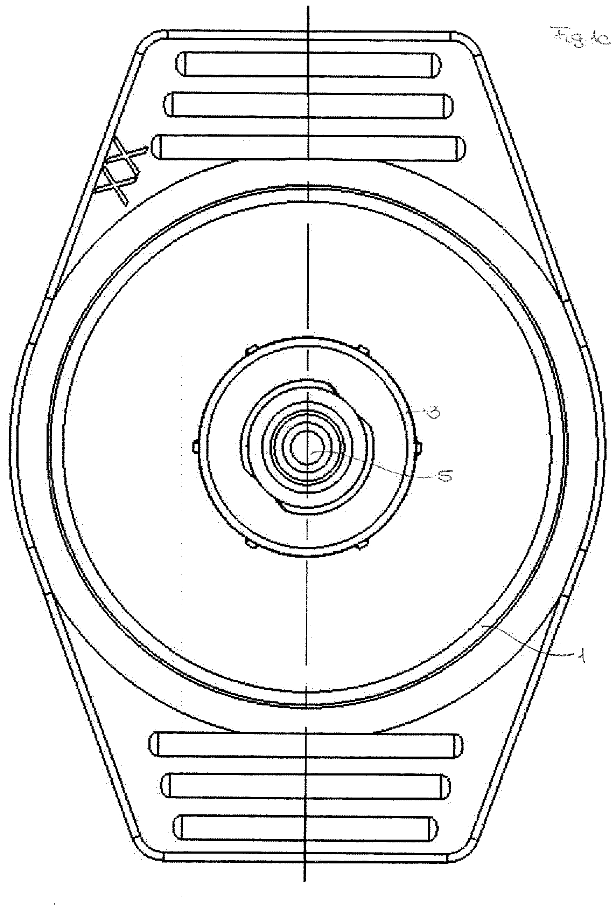

Figur 1 zeigt einen Ausschnitt, eines Spritzenkörpers 1, der einen Spritzenkonus 3 umfasst, mit einer distalen Öffnung 5. Es ist vom gesamten Spritzenkörper 1 nur der obere Teil der Spritze im Bereich des Spritzenkonus 3 gezeigt. Der Spritzenkörper 1 besteht aus einem harten Kunststoff bspw. einem Cycloolefincopolymere (COC) oder einem Cycloolefinpolymere (COP). Um den Spritzenkonus 3 ist ein Anschluss 10 für Konnektoren bspw. Aufsätze von Spritzen angeordnet. Bei dem Anschluss 10 handelt es sich vorliegend um einen Aufsatz mit einem Innengewinde 12, ohne hierauf beschränkt zu sein. Der Anschluss 10 ist vorliegend gegenüber dem Spritzenkörper 3 als eigenständiges Bauteil ausgebildet. Der Spritzenkörper als erstes Bauteil und der Anschluss als zweites Bauteil sind in den dargestellten Ausführungsformen gemäß Figur 1 lediglich mechanisch miteinander verbunden (nicht erfindungsgemäß), bspw. ist der Anschluss 10 auf den Spritzenkörper aufgesetzt und in einer Hinterschneidung 14 verrastet, d.h. der Anschluss wird form- schlüssig gehalten. Das separate Bauteil 10 kann entweder in die Hinterschneidung 14 eingedreht oder aufgepresst sein. Um das Bauteil 10 besser auf dem Spritzenkörper 1 zu halten, kann vorgesehen sein, dass der Bereich des Anschlusses umspritzt wird, bspw. mit einem Kunststoff. Durch die Umspritzung wird der Anschluss des Bauteiles 10 am Spritzenkörper stabilisiert. Das Material des Anschlusses 10 ist gemäß der Erfindung weicher als das Material des Spritzenkörpers. Bevorzugt beträgt der E-Modul des Spritzenkörpers aus einem harten Material 2500-3500 MPa der E-Modul des Anschlusses aus einem weichen Material 1200-2500 MPa. Generell ist der E-Modul des weicheren Materials des Anschlusses 10 um 10 bis 60 % geringer als der E-Modul des harten Materials des Spritzenkörpers. Während als Material des Spritzenkörpers ein Cycloolefincopolymere oder Cycloolefinpolymer eingesetzt wird, handelt es sich bei dem weichen Material des Anschlusses um ein thermoplastisches Elastomer (TPE) oder ein Elastomer oder. Polypropylen (PP) oder Polyethylen (PE). Eine Kerbschlagfestigkeit (Charpy notched impact strength) von mehr als 2kJ/m² zeichnet die angegebenen weichen Materialien aus. Figur 1c ist eine Draufsicht auf den Spritzenkörper 1. Gleiche Bauteile wie in Figur 1a - 1b tragen gleiche Bezugsziffern. Harte Materialien wie Cycloolefinpolymere (COP) oder Cycloolefincopolymere (COC) haben eine geringe Kerbschlagfertigkeit von weniger als 2kJ/m².

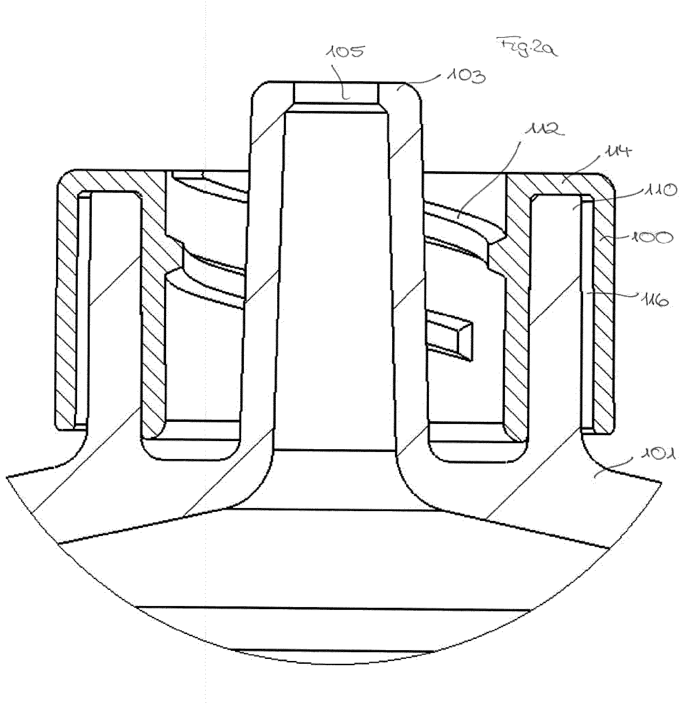

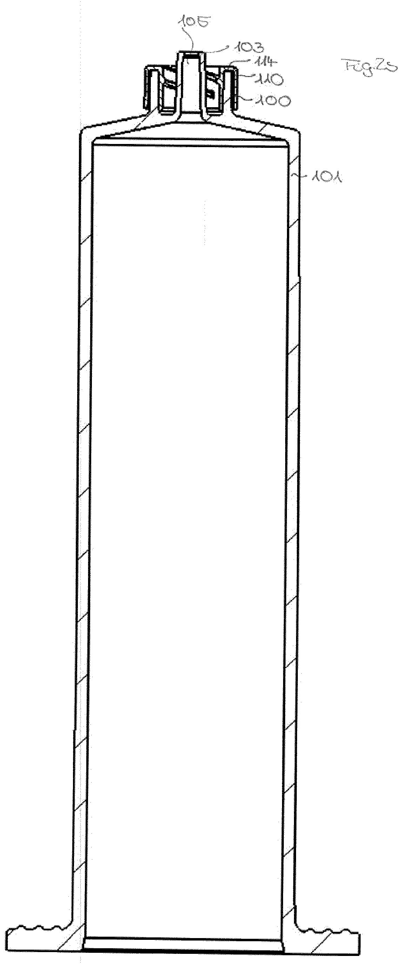

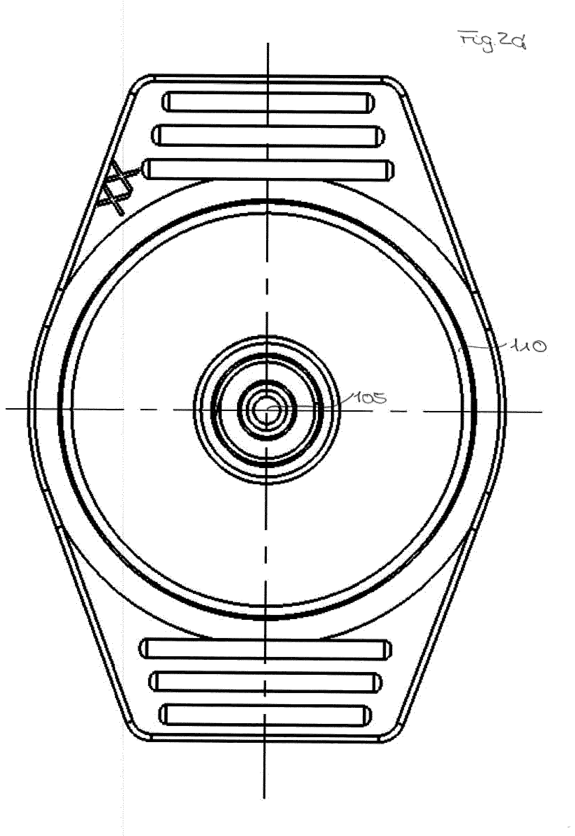

In Figur 2a-2c ist eine alternative Ausführungsform einer mechanischen Verbindung (nicht erfindungsgemäß) eines Anschlusses 100 auf einem Spritzen körper 101 gezeigt. Hierbei zeigt Figur 2a das Anschlussstück 100 mit dem Innengewinde 112. Das Anschlussstück 100 besteht aus einem weicheren Material z.B. einen thermoplastischen Elastomer, einen Elastomer, Polycarbonat, Polypropylen oder Polyethylen verglichen mit dem harten Material des Spritzenkörpers der COC oder COP sein kann. Das Anschlussstück 100 zeichnet sich dadurch aus, dass neben dem Innengewinde 112 es einen umlaufenden Aufsatz 114 mit einem Hohlraum 116 aufweist, wobei in dem Hohlraum 116 Vorsprünge oder Erhebungen 110 des Spritzenkörpers 101, eingreifen können. Durch Pressung des Anschluss kann dieses über den Hohlraum 116 auf dem Spritzenkörper 101 gehalten werden. Die detaillierte Ausgestaltung des Anschlussstückes 100 des Spritzenkörpers ist in Figur 2a gezeigt. Der Spritzenkörper 101 wie in Figur 2b gezeigt, besteht aus einem harten Material, bspw. COC und weist einen Spritzenkonus 103 mit einer distalen Öffnung 105 auf. Neben dem Spritzenkonus 103 weist der Spritzenkörper 101 eine umlaufende Erhebung 110 auf. In die umlaufende Erhebung 110 greifen die Hohlräume 116 des Anschlussstückes 100 gemäß Figur 2a ein und werden z.B. durch Einpressen nach dem Aufsetzen des Anschlussstückes auf die Erhebungen 110 gehalten. Figur 2b zeigt den kompletten Spritzenkörper 101 mit aufgesetztem Anschlussstück 110. Gleiche Bauteile wie in Figur 2a tragen dieselben Bezugsziffern. Dies trifft auch auf die Draufsicht gemäß Figur 2c zu.

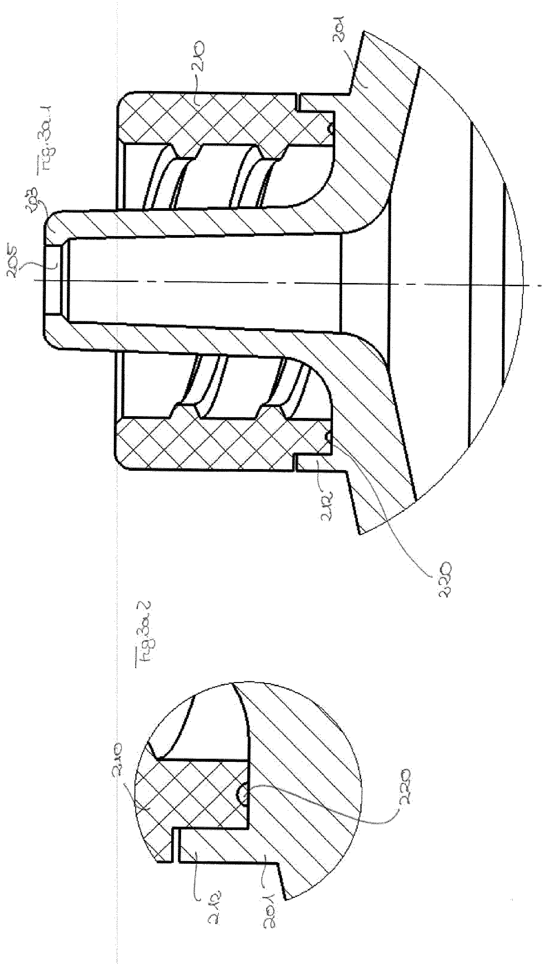

Figur 3a1 bis 3c zeigt, gemäß der Erfindung, die Verbindung des Anschlusses mit dem Spritzenkörper durch Materialschluss, bspw. durch Verschweißen. Figur 3a1 zeigt eine Ansicht eines Spritzenkörpers 201 gemäß der Erfindung mit einem auf dem Spritzenkörper 201 angebrachten Anschlussstück 210. Bei dem Spritzenkörper der Figur 3a handelt es sich um eine Kunststoffspritze aus einem harten Material, bspw. Cycloolefincolymere (COC) oder Cycloolefinpolymere (COP). Der Spritzenkörper 201 umfasst wiederrum eine distale Spitze 203 mit einer distalen Öffnung 205. Um die distale Spitze 203 herum ist das Anschlussstück 210 mit einem Innengewinde 212 zum Einschrauben eines Konnektors angeordnet. Wie in den vorausgegangenen Ausführungsformen besteht das Anschlussstück 210 aus einem weicheren Material als der Spritzenkörper 201 bspw. aus Polyethylen oder Polypropylen oder COC-E. Das weiche material kann auch abschnittsweise verwandt werden. Dann wird es bevorzugt dort eingesetzt, wo beim Einschrauben der Konnektoren hohe Kräfte auftreten.

In Figur 3a2 ist detailliert der Bereich aus Figur 3a1 dargestellt, der die Verbindung zwischen dem separaten Anschlussstück 210 und dem Spritzenkörper 201 zeigt. Diese Verbindung kann punktuell oder über die komplette Auflagefläche erfolgen. Das aus einem weichen Material bestehende separate Anschlussstück 210 wird mit Vorsprüngen 212 des Spritzenkörpers zur Anlage gebracht. Nachdem das separate Anschlussstück 210 auf den Spritzenkörper aufgesetzt wurde, wird das Anschlussstück 210 mit dem Spritzenkörper stoffschlüssig d.h. materialschlüssig verbunden, bspw. durch Schweißen, insbesondere durch Ultraschall-Schweißen. Für das Ultraschallschweißen umfassen das Anschlussstück 210 oder der Spritzenkörper 201 Materialvorhaltungen 220 besitzt, die mit einer Sonotrode zum Schwingen und damit zum Verschmelzen gebracht werden. Das Ultraschallschweißen kann punktuell oder flächig erfolgen. Wird mit Materialvorhaltungen 220 gearbeitet, so erfolgt die Verschweißung punktuell. Beim erwärmten Ultraschallschweißen ist die Auswahl der Materialien, insbesondere Ihre Kombination wichtig. Liegen die Schmelzpunkte zu weit auseinander so ist eine Verbindung der unterschiedlichen Materialien nicht mehr möglich.

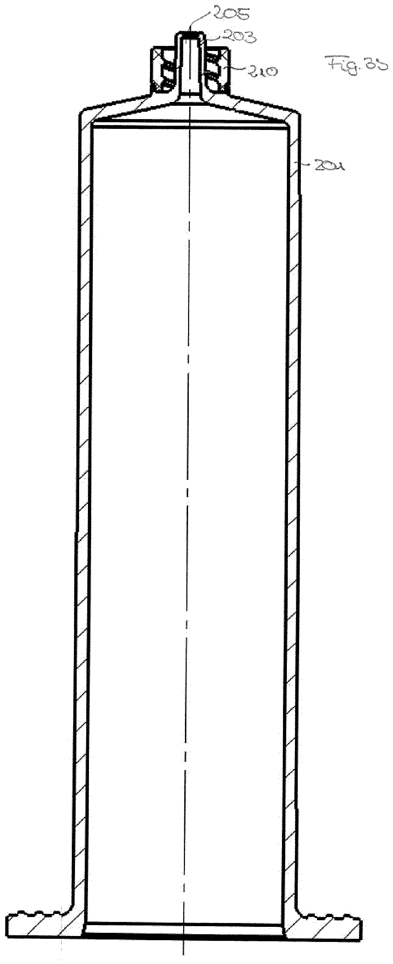

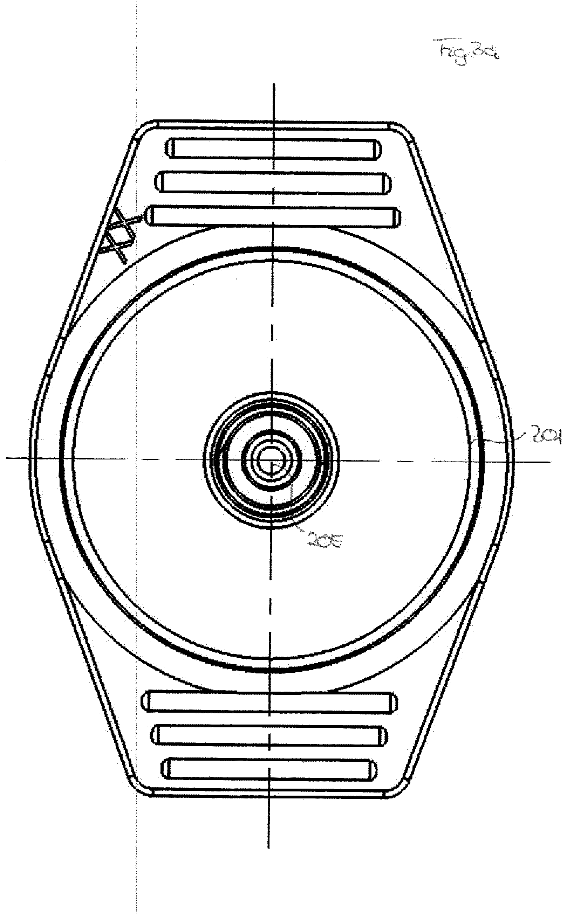

In Figur 3b ist nochmals sehr detailliert der gesamte Spritzenkörper 210 mit ultraschallverschweißtem Anschlussstück 210 gezeigt. Figur 3c zeigt eine Draufsicht auf die Ausführung gemäß Figur 3a1 - 3a2. Gleiche Bauteile sind mit denselben Bezugsziffern gekennzeichnet.

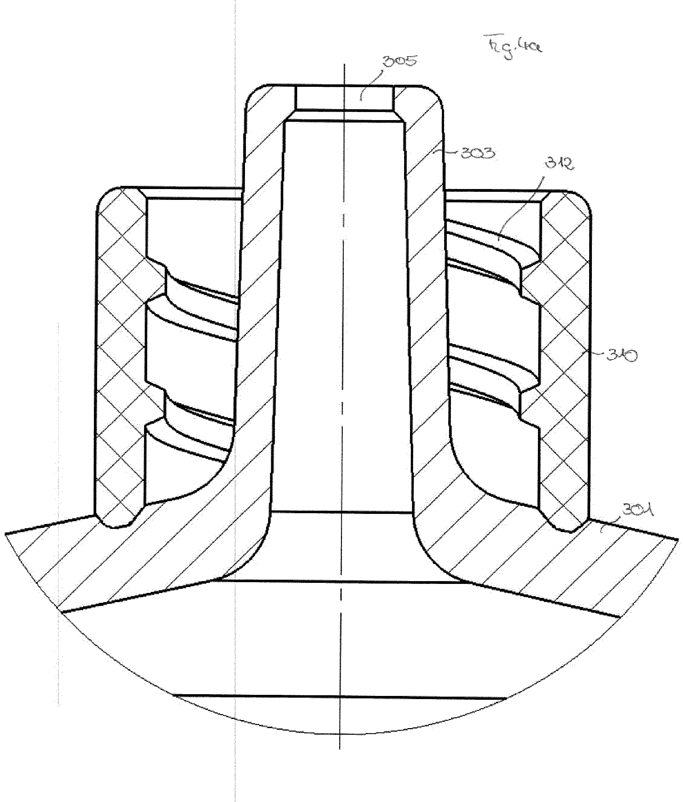

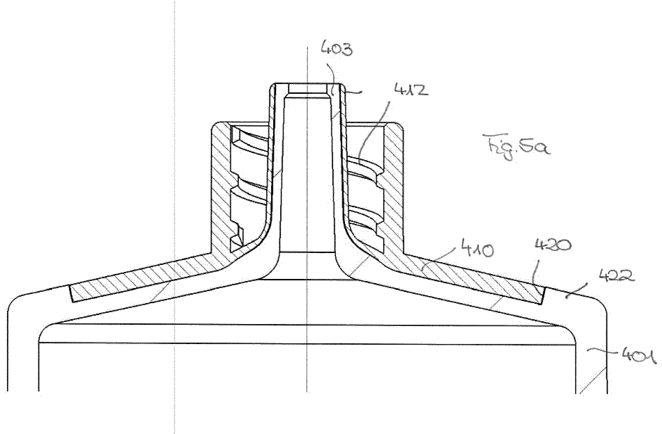

In einer alternativen Ausgestaltung der Erfindung kann anstelle des Verbindens des Anschlussstückes mit dem Spritzenkörper durch Schweißen, insbesondere Ultraschall-Schweißen der komplette Spritzenkörper mit Anschlussstück durch ein Mehrkomponenten-Spritzgieß-Verfahren hergestellt werden. Dies ist in den Figuren 4a - 4b für eine erste Ausführungsform und in Fig. 5a - 5d für eine zweite Ausführungsform gezeigt.

Figur 4a zeigt den mit Mehrkomponenten-Spritzgießen hergestellten Spritzenkörper 301. Der Spritzenkörper 301 umfasst wiederrum ein Anschlussstück 310 im Bereich des Spritzenkonus 303 mit einem Innengewinde 312 aus einem weicheren Material als der Spritzenkörper 301. Das weichere Material ist wie zuvor angegeben über den E-Modul charakterisiert.

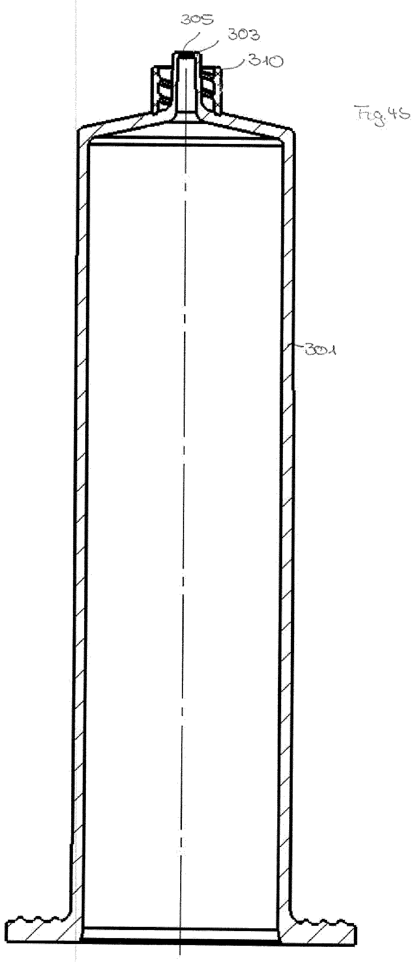

Figur 4b zeigt eine Gesamtansicht der Spritze aus Figur 4a. Das Material des Spritzenkörpers 301 ist ein hartes Kunststoffmaterial bspw. COC oder COP, wohingegen das Material des Anschlussstückes 310 ein weiches Material, bspw. Polypropylen oder Polyethylen oder COC-E ist. Gut zu erkennen in Figur 4a ist auch das Innengewinde des Anschlussstückes 312. Gleiche Bauteile wie in Figur 4a sind in Figur 4b mit denselben Bezugsziffern belegt.

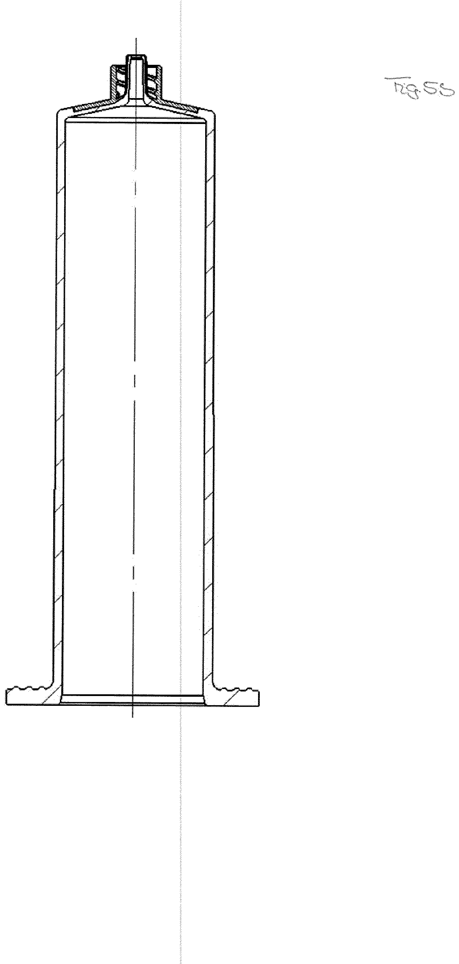

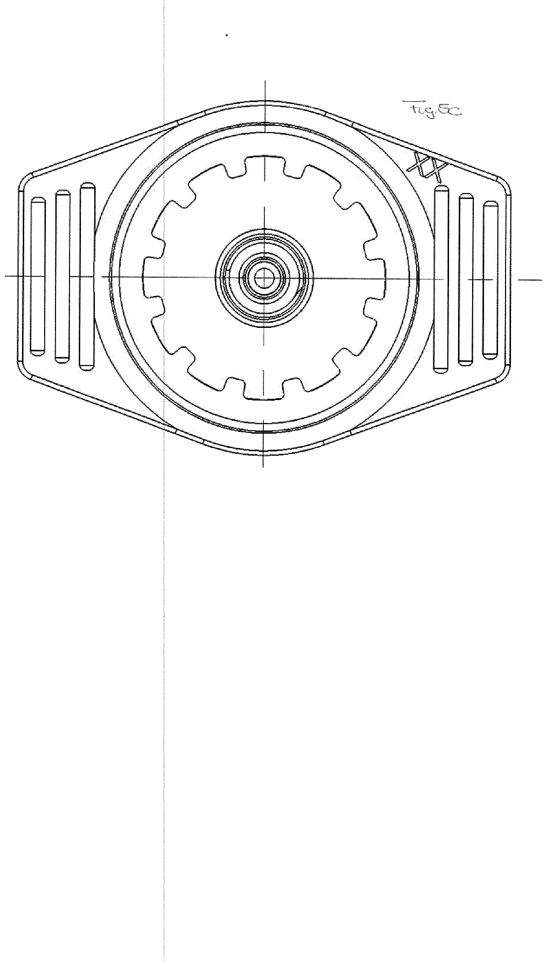

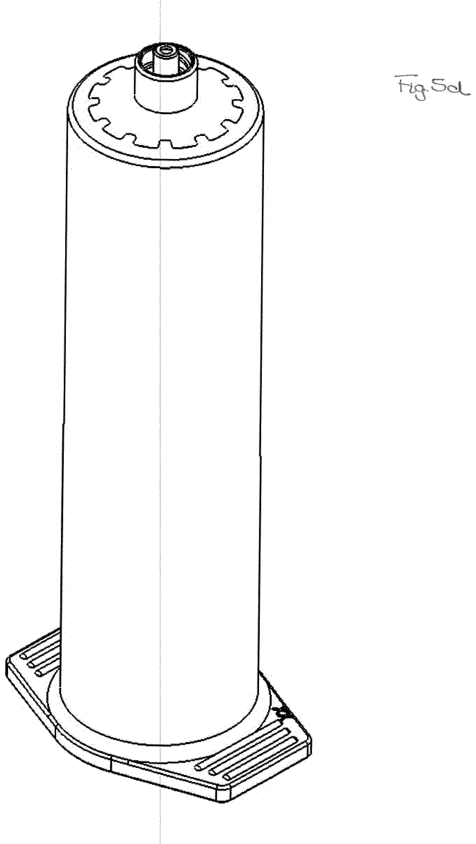

In den Figuren 5a bis 5d ist eine zweite Ausführungsform eines mit Mehrkomponenten Spritzgießen hergestellten Spritzenkörpers 401 gezeigt. Das Anschlussstück 410 im Bereich des Spritzenkörpers 401 besteht wieder aus einem weicheren Material. Das weichere Material weist einen um 10-60 % geringeren E-Modul auf, als das harte Material des Spritzenkörpers auf Charakteristisch für die zweite Ausführungsform ist, dass der Spritzenkörper 401 eine umlaufende Aufnahme 420 im Bereich des Deckels 421 des Spritzenkörpers aufweist. Das Anschlussstück aus einem weichen Material greift in die Aufnahme ein. Bei den Varianten gemäß Figur 5a bis 5d wird eine größere Kontaktfläche zur Verfügung gestellt, die eine bessere Haftung der Materialien aufeinander zulässt. Zudem zeigt die Ausführung auch eine Anbindung des weichen Materials im Konusbereich die einen Abbau der Kraftspitzen, die dort auftreten bewirkt. Des Weiteren umschließt das weiche Material des Anschlussstückes auch den Spritzenkonus 403 der Spritze 400 an der Außenseite 405. Gut zu erkennen ist auch das Gewinde 412. Fig. 5b zeigt die gesamte Spritze mit Anschlussstück 410, Figur 5c die Draufsicht und Figur 5d eine dreidimensionale Aussicht. Gleiche Bauteile wie in Figur 5a sind mit denselben Bezugsziffern belegt.

Beim Mehrkomponenten-Spritzgießen wird das gesamte Spritzgussteil aus zwei unterschiedlichen Kunststoffen hergestellt. Hierfür umfasst die Spritzgrieß-Maschine zwei Spritzeinheiten, wobei aber nur eine Schließeinheit benötigt wird. Das mit einem Mehrkomponenten-Spritzgieß-Verfahren hergestellte Bauteil gemäß Figur 4a-5d kann kostengünstig mit nur einem Werkzeug, in nur einem Arbeitsvorgang hergestellt werden. Zudem sind Geometrien umsetzbar, die bei einer Montage nicht möglich wären.

Mit der Erfindung wird erstmals ein Spritzenkörper zur Verfügung gestellt, bei dem es möglich ist, eine Vielzahl unterschiedlicher Konnektoren mit einem Anschlussstück zu verbinden, wobei das Eindrehmoment in einem breiten Bereich schwanken kann, ohne dass das Gewinde die mechanischen Belastungen an den Spritzenkörper weitergibt und dort zu Undichtigkeiten führt.

Des Weiteren sollten sämtliche Materialien sowohl des Spritzenkörpers, wie auch des Anschlussstückes Dampf-sterilisierbar sein.

Des Weiteren sind die Materialien so ausgewählt, dass sie sich nicht in Kontakt mit pharmazeutischen Medien verändern. Durch das weichere Material des Anschlussstückes wird der Reibungskoeffizient zwischen dem Konnektor und der Spritze erfüllt, so dass das Gewinde nicht überdreht werden kann.

## Patentansprüche

1. Spritze mit einem Spritzenkörper (1,101,201) sowie einem Spritzenkonus (3,103,203), der eine distale Öffnung (5) umfasst
und
einen im Bereich des Spritzenkonus angeordneten Anschluss (10,100,200), insbesondere ein Gewinde (12,112,212),
wobei der Spritzenkörper (1,101,201) aus einem ersten Material besteht und der Anschluss (10,100,200) insbesondere das Gewinde wenigstens abschnittsweise, insbesondere vollständig, aus einem zweiten Material, wobei das erste Material verschieden zum zweiten Material ist und das zweite Material ein weicheres Material als das erste Material ist, wobeidas erste Material ein Cycloolefincopolymer (COC) oder ein Cycloolefinpolymer (COP) ist,
**dadurch gekennzeichnet, dass**
der Spritzenkörper (1, 101, 201) ein erstes Bauteil der Spritze ausbildet und der Anschluss (10,100,200), insbesondere das Gewinde (12,112,212), ein zweites Bauteil und erstes und zweites Bauteil stoffschlüssig miteinander verbunden sind.

2. Spritze nach Anspruch 1,
**dadurch gekennzeichnet, dass**,
das erste Material einen E-Modul hat und das zweite Material einen E-Modul hat der um 10 bis 60 % geringer ist als der E-Modul des ersten Materials.

3. Spritze nach einem der Ansprüche 1 bis 2,
**dadurch gekennzeichnet, dass**
der E-Modul des ersten Materials im Bereich >2500 bis 3500 MPa, bevorzugt 2700 bis 3200 MPa und der E-Modul des zweiten Materials 1200 bis 2500 MPa, bevorzugt 1500 bis 1800 MPa liegt.

4. Spritze nach einem der Ansprüche 1 bis 3,
**dadurch gekennzeichnet, dass**
die Kerbschlagfestigkeit des zweiten, weichen Materials größer als 2kJ/m² ist.

5. Spritze nach einem der Ansprüche 1 bis 4,
**dadurch gekennzeichnet, dass**
das zweite Material ein thermoplastisches Elastomer (TPE), ein Elastomer, Polypropyen (PP), Polycabonat (PC), Polyethylen (PE), Polyamid (PA) oder COC-E ist.

6. Spritze nach einem der Ansprüche 1 bis 5,
**dadurch gekennzeichnet, dass**
das erste und das zweite Material Materialien umfasst, die bei Temperaturen > 100° C, insbesondere > 121° C, insbesondere maximal 180° C, sterilisierbar sind.

7. Spritze nach einem der Ansprüche 1 bis 6,
**dadurch gekennzeichnet, dass**
der Anschluss (10, 100, 200) eine Außengeometrie aufweist und die Außengeometrie der Außengeometrie eines Luer-Lock-Adapters entspricht.

8. Verfahren zur Herstellung einer Spritze, umfassend nachfolgende Schritte:
- ein Spritzenkörper (1,101,201) aus einem ersten Material und
- ein Anschluss (10, 100, 200), insbesondere ein Gewinde (12, 112, 212), aus einem zweiten Material, das weicher als das erste Material ist, wird zur Verfügung gestellt, wobei das zweite Material einen E-Modul aufweist, der 10 bis 60 % geringer ist als der E-Modul der erste Materials, wobei das erste Material ein Cycloolefincopolymer (COC) oder ein Cycloolefinpolymer (COP) ist,
- der Anschluss (10, 100, 200) und der Spritzenkörper (1, 101, 201) werden stoffschlüssig miteinander verbunden
oder
die Spritze wird durch Mehrkomponenten-Spritzgießen erhalten.

9. Verfahren nach Anspruch 9,
**dadurch gekennzeichnet, dass**
Spritzenkörper und Anschluss stoffschlüssig durch Schweißen, insbesondere Ultraschweißen, miteinander verbunden werden.

10. Verfahren nach Anspruch 9,
**dadurch gekennzeichnet, dass**
das Mehrkomponenten-Spritzgießen derart geführt wird, dass der Anschluss ab einer Belastung von wenigstens 25 Ncm, insbesondere wenigstens 35 Ncm definiert lösbar ist.

## Claims

1. Syringe having a syringe body (1, 101, 201) and a syringe cone (3, 103, 203), which comprises a distal opening (5) and
a connection (10, 100, 200), in particular a thread (12, 112, 212), arranged in the region of the syringe cone,
wherein the syringe body (1, 101, 201) consists of a first material and the connection (10, 100, 200), in particular the thread, consists at least in sections, in particular completely, of a second material,
wherein the first material is different from the second material and the second material is a softer material than the first material,
wherein the first material is a cycloolefin copolymer (COC) or a cycloolefin polymer (COP),
**characterized in that**
the syringe body (1, 101, 201) forms a first component of the syringe and the connection (10, 100, 200), in particular the thread (12, 112, 212), is a second component and the first and second components are joined to one another in a materially bonded manner.

2. Syringe according to claim 1,
**characterized in that**
the first material has a modulus of elasticity and the second material has a modulus of elasticity which is 10 to 60 % lower than the modulus of elasticity of the first material.

3. Syringe according to one of claims 1 to 2,
**characterized in that**
the modulus of elasticity of the first material is in the range >2500 to 3500 MPa, preferably 2700 to 3200 MPa, and the modulus of elasticity of the second material is 1200 to 2500 MPa, preferably 1500 to 1800 MPa.

4. Syringe according to one of claims 1 to 3,
**characterized in that**
the notched impact strength of the second, soft material is greater than 2kJ/m².

5. Syringe according to one of claims 1 to 4,
**characterized in that**
the second material is a thermoplastic elastomer (TPE), an elastomer, polypropylene (PP), polycabonate (PC), polyethylene (PE), polyamide (PA) or COC-E.

6. Syringe according to one of claims 1 to 5,
**characterized in that**
the first and second materials comprise materials that can be sterilized at temperatures >100°C, in particular >121°C, in particular a maximum of 180°C.

7. Syringe according to one of claims 1 to 6,
**characterized in that**
the connector (10, 100, 200) has an outer geometry and the outer geometry corresponds to the outer geometry of a Luer-lock adapter.

8. Method for manufacturing a syringe, comprising the following steps:
- a syringe body (1, 101, 201) made of a first material; and
- providing a connector (10, 100, 200), in particular a thread (12, 112, 212), made of a second material which is softer than the first material, wherein the second material has a modulus of elasticity which is 10 to 60% lower than the modulus of elasticity of the first material, wherein the first material is a cycloolefin copolymer (COC) or a cycloolefin polymer (COP),
- the connection (10, 100, 200) and the syringe body (1, 101, 201) are joined to one another in a materially bonded manner
or
the syringe is obtained by multi-component injection molding.

9. Method according to claim 9, **characterized in that** the syringe body and the connection are joined to one another in a materially bonded manner by welding, in particular ultrasonic welding.

10. Method according to claim 9, **characterized in that** the multi-component injection molding is carried out in such a way that the connection can be released in a defined manner from a load of at least 25 Ncm, in particular at least 35 Ncm.

## Revendications

1. Seringue avec un corps de seringue (1, 101, 201) ainsi qu'un cône de seringue (3, 103, 203) qui comprend une ouverture distale (5) et un raccord (10, 100, 200) disposé dans la zone du cône de seringue, en particulier un filetage (12, 112, 212),
le corps de seringue (1, 101, 201) étant constitué d'un premier matériau et le raccord (10, 100, 200), en particulier le filetage, étant constitué au moins sur certaines parties, en particulier entièrement, d'un deuxième matériau,
le premier matériau étant différent du deuxième matériau et le deuxième matériau étant un matériau plus souple que le premier matériau,
le premier matériau étant un copolymère de cyclooléfine (COC) ou un polymère de cyclooléfine (COP),
**caractérisée en ce que**
le corps de seringue (1, 101, 201) forme un premier composant de la seringue et le raccord (10, 100, 200), en particulier le filetage (12, 112, 212), un deuxième composant et le premier et le deuxième composant sont reliés entre eux avec apport de matière.

2. Seringue selon la revendication 1,
**caractérisée en ce que**
le premier matériau a un module d'élasticité et le deuxième matériau a un module d'élasticité qui est inférieur de 10 à 60 % au module d'élasticité du premier matériau.

3. Seringue selon l'une des revendications 1 à 2,
**caractérisée en ce que**
le module d'élasticité du premier matériau est compris dans la plage >2500 à 3500 MPa, de préférence de 2700 à 3200 MPa et le module d'élasticité du deuxième matériau est compris dans la plage de 1200 à 2500 MPa, de préférence de 1500 à 1800 MPa.

4. Seringue selon l'une des revendications 1 à 3,
**caractérisée en ce que**
la résistance au choc sur entaille du deuxième matériau souple est supérieure à 2kJ/m².

5. Seringue selon l'une des revendications 1 à 4,
**caractérisée en ce que**
le deuxième matériau est un élastomère thermoplastique (TPE), un élastomère, du polypropylène (PP), du polycabonate (PC), du polyéthylène (PE), du polyamide (PA) ou du COC-E.

6. Seringue selon l'une des revendications 1 à 5,
**caractérisée en ce que**
le premier et le deuxième matériau comprennent des matériaux qui sont stérilisables à des températures > 100 °C, en particulier > 121 °C, en particulier au maximum à 180 °C.

7. Seringue selon l'une des revendications 1 à 6,
**caractérisée en ce que**
le raccord (10, 100, 200) présente une géométrie extérieure et la géométrie extérieure correspond à la géométrie extérieure d'un adaptateur Luer-Lock.

8. Procédé de fabrication d'une seringue, comprenant les étapes suivantes :
- un corps de seringue (1, 101, 201) constitué d'un premier matériau et
- un raccord (10, 100, 200), en particulier un filetage (12, 112, 212), constitué d'un deuxième matériau qui est plus souple que le premier matériau, sont mis à disposition, le deuxième matériau présentant un module d'élasticité qui est inférieur de 10 à 60 % au module d'élasticité du premier matériau, le premier matériau étant un copolymère de cyclooléfine (COC) ou un polymère de cyclooléfine (COP),
- le raccord (10, 100, 200) et le corps de seringue (1, 101, 201) sont reliés entre eux avec apport de matière
ou
la seringue est obtenue par moulage par injection à plusieurs composants.

9. Procédé selon la revendication 9,
**caractérisé en ce que**
le corps de seringue et le raccord sont reliés entre eux avec apport de matière par soudage, en particulier par soudage par ultrasons.

10. Procédé selon la revendication 9,
**caractérisé en ce que**
le moulage par injection à plusieurs composants est réalisé de telle sorte que le raccord est détachable de manière définie à partir d'une charge d'au moins 25 Ncm, en particulier d'au moins 35 Ncm.
